# EUROPÄISCHE PATENTSCHRIFT

(11) **EP 0 554 279 B1**
(45) Veröffentlichungstag und Bekanntmachung des Hinweises auf die Patenterteilung: **05.10.1994**
(21) Anmeldenummer: 91917735.2
(22) Anmeldetag: 16.10.1991
(51) Int. Cl.: A61B 5/00

(54) **MESSSONDE**
MEASURING PROBE
TETE DE MESURE

(30) Priorität: 25.10.1990 DE 4034019
(43) Veröffentlichungstag der Anmeldung: 11.08.1993
(73) Patentinhaber: FRAUNHOFER-GESELLSCHAFT ZUR FÖRDERUNG DER ANGEWANDTEN FORSCHUNG E.V., 80636 München (DE)
(72) Erfinder: BOLLEROTT, Michael, D-4300 Essen 16 (DE)
(74) Vertreter: Münich, Wilhelm, Dr.
(86) Internationale Anmeldenummer: DE9100812
(87) Internationale Veröffentlichungsnummer: WO9207505

(56) Entgegenhaltungen:
- WO-A-84/01688
- DE-A- 2 632 899
- DE-A- 3 219 558
- FR-A- 2 290 874
- US-A- 4 075 632
- US-A- 4 528 987

## Beschreibung

### Technisches Gebiet

Die Erfindung bezieht sich auf eine Meßsonde mit einer Empfangseinheit, die ein von einer beabstandeten Sende- und Auswerteeinrichtung abgestrahltes Hochfrequenzfeld empfängt, und mit einer Energieversorgungseinheit, die aus dem hochfrequenten Ausgangssignal der Empfangseinheit eine Versorgungsspannung für die Meßsonde generiert,
Derartige Meßsonden werden für die verschiedensten telemetrischen Messungen von physikalischen oder chemischen Meßgrößen verwendet. Eine beispielhafte Anwendung ist die Messung der Körpertemperatur oder die Identifikation von Lebewesen, beispielsweise von Tieren in einer größeren Herde.

### Stand der Technik

Meßsonden mit einer Empfangseinheit, die ein von einer beabstandeten Sende-und Auswerteeinrichtung abgestrahltes Hochfrequenzfeld empfängt, und mit einer Energieversorgungseinheit, die aus dem hochfrequenten Ausgangssignal der Empfangseinheit eine Versorgungsspannung für die Meßsonde generiert, sind beispielsweise aus der DE-A-32 19 558, FR-A-2 290 874 oder der US-PS 4 075 632 bekannt. Aufgrund dieser Ausbildung benötigen diese Meßsonden zur Stromversorgung kein eigenes Netzteil bzw. keine eigene Batterie oder einen eigenen Akkumulator.

Ferner weisen die aus den vorgenannten Druckschriften bekannten Meßsonden eine Sensoreinheit auf, die ein analoges und bei der aus der US-PS 4 075 632 bekannten Meßsonde zusätzlich ein digitales Signal erzeugt, das eine Sendeeinheit an eine stationäre Empfangs- und Auswerteeinheit überträgt. Bei der aus der DE-A-32 19 558 bekannten Meßsonde setzt ein spannungsgesteuerter Oszillator das Signal des einzigen Sensors in ein Signal um, dessen Frequenz proportional dem Ausgangssignal ist, während bei der aus der US-PS 4 075 632 bekannten Meßsonde mehrere Modulationstransistoren vorgesehen sind. Mehrere Modulationseinheiten modulieren das hochfrequente Ausgangssignal der Empfangseinheit mit dem bzw. den Ausgangssignalen und legen das modulierte Signal an die Sendeeinheit an.

Diese bekannten Meßsonden haben damit zwar den Vorteil, daß sie auch noch nach Jahren benutzt werden können, da sie die gesamte Energie, die sie benötigen, von außen über ein Hochfrequenzfeld beziehen.

Nachteilig ist jedoch, daß keine direkte Kalibrierung der Meß- und Empfangsgeräte durchgeführt werden kann, da die Absolutfrequenz der Sonde durch den verwendeten Oszillator und die angewandte CMOS-Technologie stark streut (etwa 30-40% Streuung) und sich darüberhinaus in Folge von Schwankungen der Versorgungsspannung ändern kann. Hierbei ist weiter zu berücksichtigen, daß bei derartigen Meßsonden die Versorgungsspannung aufgrund von Schwankungen der Feldstärke des Hochfrequenzfeldes stark schwanken kann. Darüberhinaus ändert sich der Signal-/Störabstand aufgrund von Änderungen des Abstandes Meßsonde/Auswerteeinrichtung.

Weiterhin besteht keine Übermittlungsmöglichkeiten eines Identifikationscodes. Es ist daher nicht möglich, mehrere Mikromeßsonden gleichzeitig zu betreiben und ihre Signale voneinander zu unterscheiden.

### Beschreibung der Erfindung

Es ist Aufgabe der Erfindung, eine Meßsonde mit einer Empfangseinheit, die ein von einer beabstandeten Sende -und Auswerteeinrichtung abgestrahltes Hochfrequenzfeld empfängt, und mit einer Energieversorgungseinheit, die aus dem hochfrequenten Ausgangssignal der Empfangseinheit eine Versorgungsspannung für die Meßsonde generiert, derart weiterzubilden, daß eine Eichung der Meßsignale bei annähernd konstantem Signal-/Störabstand und zusätzlich die Identifikation der einzelnen Meßsonden bzw. des jeweils übertragenen Signals möglich ist.

Diese Aufgabe wird erfindungsgemäß durch die kennzeichnenden Merkmale des Anspruchs 1 gelöst.

Hierdurch ist es möglich, zu erkennen, ob eine Frequenzänderung durch Schwankungen der Versorgungsspannungen oder durch eine Änderung des Sensorausgangssignals hervorgerufen wird.

Weiterhin ist der Modulationshub der einzigen Modulationseinheit programmierbar, die das hochfrequente Ausgangssignal der Empfangseinheit mit dem Ausgangssignal des Oszillators moduliert, und das modulierte hochfrequente Signal an die Sendeeinheit anlegt. Durch diese Maßnahme kann auch bei sich änderndem Abstand der Meßsonde von der Auswerteeinrichtung der Signal-/Störabstand annähernd konstant gehalten werden. Der programmierbare Modulationshub ermöglicht es darüberhinaus bei einer monolithischen Integration des Modulators eine Anpassung an Einsatzbedingungen ohne Neudimensionierung der Schaltung.

Durch die Identifikationseinheit, die einen Identifikationscode erzeugt, mit dem das hochfrequente Signal zur Identifikation der Sonde modulierbar ist, deren Signal jeweils übertragen wird, ist es möglich beim Einsatz von mehreren Meßsonden eine Unterscheidung vorzunehmen, welche Meßsonde welche Größe oder Größen mißt bzw. welches Ausgangssignal "gerade" übertragen wird.

Durch die Verwendung eines Multiplexers ist es weiterhin möglich, eine Eichung von externen Meßgeräten vorzunehmen, die das von der Mikromeßsonde ausgesandte Signal auffangen und verarbeiten.

Die erfindungsgemäße Meßsonde kann damit zur hochgenauen Messung beliebiger physikalischer oder chemischer Größen und darüberhinaus auch zur Identifikation des Meßobjekts eingesetzt werden: Dies ist zum Beispiel der Fall, wenn die Mikromeßsonde zur Identifikation von Tieren, die in großen Herden zusammenleben, oder die sehr selten sind, dienen soll. Die Sonde wird dabei dem Tier durch eine Injektion eingesetzt oder durch eine kleine Operation eingepflanzt. Die Sonde ermöglicht eine eindeutige, kaum entfernbare Kennzeichnung der Tiere und stellt darüberhinaus eine Art Diebstahlsicherung dar, da sich die Meßsonde nur durch eine Operation wieder entfernen läßt.

Weiterbildungen der Erfindung sind in den Unteransprüchen angegeben:
Gemäß Anspruch 2 sind mehrere Sensoren vorgesehen, so daß mehrere Meßwerte erfaßt und nacheinander übertragen werden können.

Nach Anspruch 3 weist die Identifikationseinheit eine Steuerlogik und einen Speicher auf, in dem die Identifikationscodes gespeichert sind, die die Steuereinheit gesteuert durch ein Steuersignal des Multiplexers ausliest, und an den Oszillator anlegt.

Um den Identifikationscode einmal einzugeben, wird gemäß Anspruch 4 eine Programmiereinheit mit der Meßsonde verbunden.

Nach Anspruch 5 überträgt die Steuereinheit den seriellen, Biphase-kodierten Datenstrom an den Oszillator, der aus diesem Datenstrom ein FSK-Signal generiert.

Der im Anspruch 6 gekennzeichnete Oszillator mit einem Fensterkomparator hat den Vorteil, daß seine Frequenz weitgehend unabhängig von Schwankungen der Versorgungsspannung ist.

Weiterhin ist es von Vorteil, wenn gemäß Anspruch 7 die Empfangs- und die Sendeeinheit denselben Schwingkreis verwenden.

Weitere vorteilhafte Weiterbildungen sind in den Ansprüchen 8 bis 11 angegeben.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: den prinzipiellen Aufbau einer Meßeinrichtung, bei der eine erfindungsgemäße Meßsonde verwendbar ist,
- Fig. 2: den Aufbau einer erfindungsgemäßen Meßsonde mit Referenzsignal und programmierbarem Identifikationscode, und
- Fig. 3: einen erfindungsgemäß verwendeten Oszillator mit Fensterkomparator.

### Beschreibung eines Ausführungsbeispiels

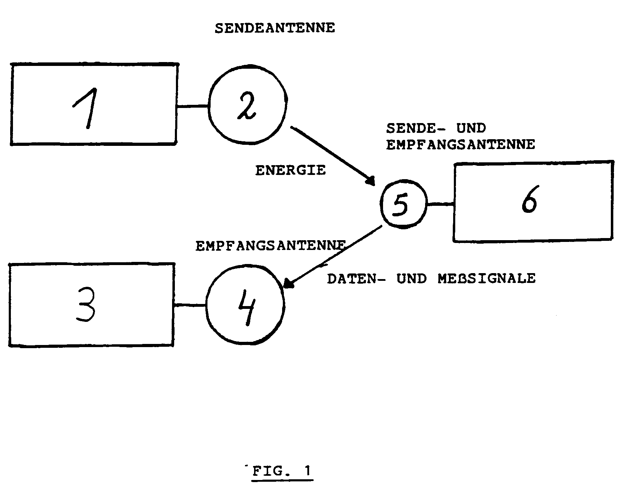

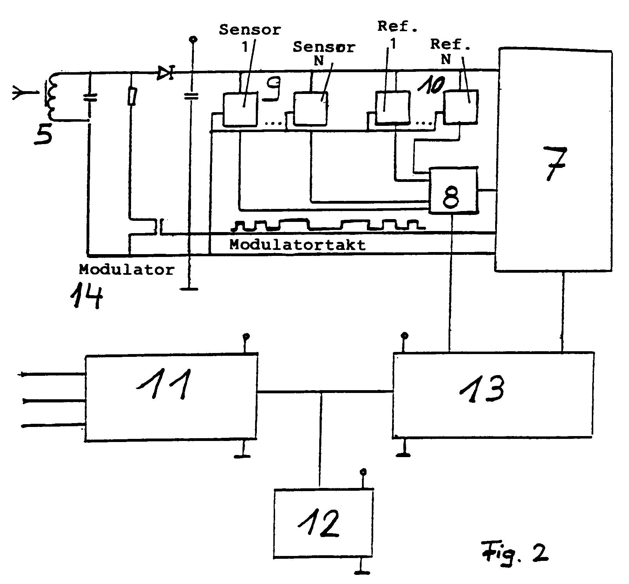

Fig. 1 zeigt den prinzipiellen Aufbau einer Meßeinrichtung, bei der eine erfindungsgemäße Meßsonde verwendbar ist. Die Meßeinrichtung weist einen Hochfrequenzsender 1 und eine Sendeantenne 2 sowie eine Empfangs- und Auswerteelektronik 3 und eine Empfangsantenne 4 auf. Die eigentliche Meßsonde weist eine Sende- und Empfangsantenne 5 und einen Steuer- und Meßteil 6 auf, der in Fig. 2 im einzelnen dargestellt ist:

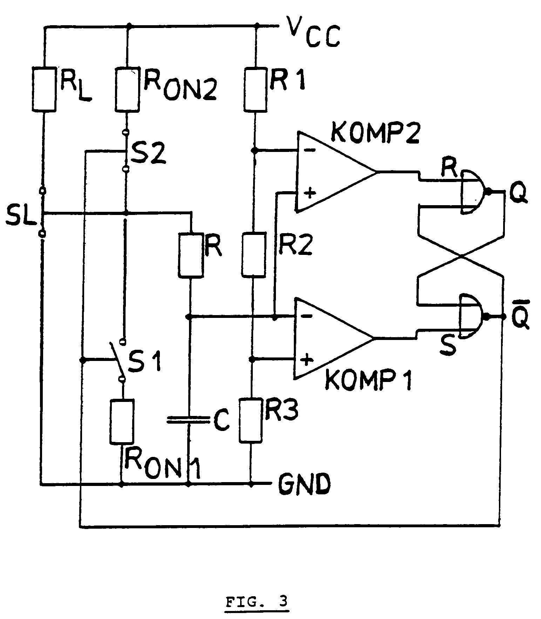

Fig. 2 zeigt, daß ein Oszillator 7, der seine Frequenz proportional zur Änderung eines noch näher beschriebenen Sensorsignals ändert, einen Modulator 8 treibt, der in Abhängigkeit von der Oszillatorfrequenz eine Amplitudenmodulation eines Hochfrequenzträgersignals durchführt. Der Oszillator 7 bezieht seine Energie aus dem Hochfrequenzträgersignal, das er selber anschließend moduliert. Der Oszillator muß daher so aufgebaut sein, daß die Oszillatorfrequenz weitgehend unabhängig von der Betriebsspannung ist. Eine Ausführungsform eines derartigen Oszillators wird nachstehend in Verbindung mit Fig. 3 beschrieben.

Mit dem Eingangsanschluß des Oszillators 7 werden über einen Multiplexer 8 die Ausgangsanschlüsse wenigstens eines Sensor, bei dem gezeigten Ausführungsbeispiel von Sensoren 9₁ ...9_{N}, und die Ausgangsanschlüsse von wenigstens einem, einem oder mehreren Sensoren zugeordneten Referenzelement, bei dem gezeigten Ausführungsbeispiel von mehreren Referenzelementen 10₁...10_{N}, die beispielsweise Widerstände sein können, verbunden. Damit lassen sich auf einfache Weise eine oder mehrere Referenzfrequenzen erzeugen, die zum Kalibrieren von externen Meßgeräten und/oder zur Erzeugung des Identifikationscodes sowie zur Regelung der von dem Hochfrequenzsender abgestrahlten Leistung benutzt werden können.

Selbstverständlich können anstelle von Widerständen als Sensor- und Referenzelemente auch andere Bauteile, wie zum Beispiel Kapazitäten oder auch aktive Bauelemente verwendet werden. Der Oszillator 7 muß dann nur so dimensioniert werden, daß er die Änderung dieses oder dieser Bauteile in eine Frequenzänderung umsetzt.

Die in Fig. 2 dargestellte Schaltung kann auch ohne oder mit nur einem Sensor betrieben werden, wenn die Genauigkeitsanforderungen gering sind, und der Abstand zwischen Sende-/Empfangseinheit und Transponder nur geringe Schwankungen aufweist. Ohne Sensor dient die Schaltung zur reinen Identifikation eines Objekts. Die Schaltung ist so konstruiert, daß sie auch in diesem speziellen Anwendungsfall einwandfrei funktioniert. Dies ist aber nur ein Spezialfall des normalen Einsatzes, bei dem wenigstens ein Sensor verwendet wird.

Normalerweise sollen ein oder mehrere Sensorsignale mit hoher Genauigkeit gemessen werden, beispielsweise soll ein Temperatursensor die Temperatur auf ± 0,1°C genau messen. Dies ist mit dieser Schaltung möglich. Zusätzlich kann der Abstand zwischen Sende-/Empfangseinheit und Transponder erheblichen Schwankungen unterworfen sein. In diesen beiden Fällen ist ein Referenzelement notwendig, welches keine externen Größen, wie die Körpertemperatur, mißt, sondern das den internen Zustand der Meßsonde wiedergibt. Die Parameter, die hierbei in Betracht kommen, sind z.B. die Betriebstemperatur oder die Betriebsspannung des Chips bzw. der Schaltung. Wird z.B. die Betriebsspannung gemessen und zusätzlich zu dem eigentlichen Sensorsignal mit übertragen, dann besteht die Möglichkeit, einen geschlossenen Regelkreis aufzubauen. Es muß dazu nur eine Kennlinie aufgenommen werden, die den Zusammenhang zwischen der an die Schaltung angelegten Betriebsspannung und der durch das/die Referenzelement/e 10 und den Oszillator 7 in Fig. 2 erzeugten Frequenz wiedergibt.

Selbst ein Oszillator wie der nachstehend in Verbindung mit Fig. 3 beschriebene Fensterkomparator, der aufgrund seiner Schaltung eine Ausgangsfrequenz erzeugt, die weitgehend unabhängig von der Betriebsspannung ist, hat keine unendlich große Betriebsspannungsunterdrückung. Für sehr präzise Messungen von z.B. ± 0,1°C ist die Betriebsspannungsunterdrückung sogar bei weitem zu klein. Auch die Temperatur der Schaltung kann bei hochpräzisen Messungen einen zu großen Einfluß auf die Frequenz des Oszillators haben. Diese Abhängigkeit kann ebenfalls gemessen und korrigiert werden, indem in der Auswerteeinheit eine Korrekturrechnung mit der gemessenen Chiptemperatur durchgeführt wird.

Die Identifikationscodelogik besteht im wesentlichen aus zwei Teilen, einer Programmierlogik 11, mit deren Hilfe jede Mikromeßsonde einen spezifischen Code erhält, der in einem nicht flüchtigen Speicher 12, beispielsweise einem ROM, EPROM, EEPROM, Fuseable Link-Speicher, gespeichert ist, und einer Logikschaltung 13, die diesen Code aus dem Speicher 12 ausliest und in einen seriellen Datenstrom umwandelt. Die Logik, die den seriellen Datenstrom erzeugt, ist Teil der Steuerlogik.

Der Identifikationscode selbst besteht aus den Nutzdaten, Start- und Stopbits sowie Paritätsbits. Durch die Paritätsbits wird die Datensicherheit erheblich erhöht. Dies wirkt sich besonders dann aus, wenn der Signal/Störabstand im Empfänger sehr klein geworden ist, weil der Abstand zum Transponder groß ist.

Die empfangenen Daten werden in einem Standardformat, beispielsweise der V.24- bzw. RS 232 C-Norm übertragen. Dadurch können sie auf sehr einfache Weise durch einen Standardschnittstellenbaustein für serielle Schnittstellen aufbereitet und einem Mikroprozessor zur Verfügung gestellt werden.

Die Steuerlogik koordiniert das Zusammenspiel der einzelnen Schaltungsteile und erzeugt ein Datenprotokoll zur Aussendung der Meßdaten und des Identifikationscodes. Sie schaltet für eine gewisse Zeit den oder die Sensoren an den Eingangsanschluß des Oszillators, so daß während dieser Zeit die Oszillatorfrequenz proportional zum Meßsignal ist.

Damit die einzelnen Meßdaten voneinander unterschieden werden können, falls der Oszillator für unterschiedliche Sensoren bzw. Referenzelemente zufällig die gleiche Frequenz erzeugt, sollte zwischen den Meßsignalen eine kurze Pause liegen, oder aber für eine kurze Zeit ein Referenzsignal gesendet werden. Anschließend schaltet die Steuerlogik für eine definierte Zeit den oder die Referenzwiderstände an den Eingangsanschluß des Oszillators. Die Zeit, für die die Referenzwiderstände am Eingang des Oszillators anliegen, wird durch einen Taktteiler ermittelt, der die Anzahl der Schwingungen des Referenzsignals mißt. Der Identifikationscode wird übertragen, indem er zunächst in einen seriellen digitalen Biphase-Code umgesetzt wird. Die Empfangs- und Auswerteelektronik 3 kann aus dem Biphase-Code sowohl den Takt als auch die Daten sehr einfach zurückgewinnen. Der Biphase-Code wird dann im Oszillator in ein FSK-Signal umgesetzt, wobei über den Multiplexer ein Referenzelement an den Oszillator angelegt wird.

Die Frequenzen F₁ und F₂ werden über einen Taktteiler erzeugt, der durch den Biphase-Code umgeschaltet wird. Der Taktteiler gehört zum Oszillator. Bei der FSK Modulation (Frequency Shift Keying Modulation) entspricht eine logische "1" dabei einer Frequenz F1 und eine logische "0" einer Frequenz F2. Das gesamte Datenprotokoll besteht darin, daß nach einer Startkennung zunächst der Identifikationscode ausgestrahlt wird. Anschließend wird eine Stopkennung übertragen und danach wird für eine bestimmte Zeit das oder die Meßsignale ausgesandt. Ferner werden noch Parity-Bits zur Sicherung und Fehlererkennung übertragen.

Wie lange das oder die Meßsignale übertragen werden, sollte sich nach der gewünschten Meßgenauigkeit richten. Nach Beendigung eines Sendezyklusses beginnt die Steuerlogik von vorne und sendet wieder einen Daten- und Meßzyklus aus. Die Start- und Stopkennung lassen sich an dem Wert der Frequenz und der Zeitdauer, in der sie gesendet werden, erkennen. Der Identifikationscode sollte mit einer möglichst hohen Datenrate übertragen werden, damit es zum einen möglich ist, eine große Datenmenge zu übertragen und zum anderen, damit ein gesamter Zyklus nicht zu lange dauert.

Da nur ein Oszillator verwendet wird, erreicht man, daß die Betriebsspannung auf alle Signale nahezu den gleichen Einfluß hat. Dies bedeutet, daß man aus der Änderung der Referenzfrequenz den Einfluß der Betriebsspannung auf die Meßfrequenz ermitteln und dadurch auch beseitigen kann. Dies ermöglicht es, die Mikromeßsonde in einem weiten Betriebsspannungsbereich zu betreiben, was gleichzusetzen ist mit einem großen räumlichen Sende- und Empfangsbereich um die Hochfrequenzsende- und Empfangsantennen herum. Es läßt sich somit eine erhebliche Verbesserung der Genauigkeit des Meßsignals erreichen ohne die Betriebsspannungsunterdrückung der Schaltung zu erhöhen. Dies wäre nur mit erheblichem Aufwand möglich und würde bedeuten, daß die abgestrahlte Hochfrequenzleistung erheblich größer sein müßte als bei der beschriebenen Schaltung.

Um den Modulationshub zu verbessern und um die Oszillatorfrequenz unabhängiger von der Betriebsspannung zu machen, wird der Modulator 14 direkt an den Schwingkreis angeschlossen und nicht wie bisher hinter der Gleichrichterdiode.

Der Modulationshub ist ebenfalls programmierbar; dies ermöglicht eine optimale Anpassung an die Sende- und Empfangseinheit. Dadurch kann ein maximaler Signal-Störabstand erzielt werden, ohne daß die Betriebsspannung aufgrund eines zu hohen Modulationshubes zusammenbricht.

Der Modulationshub erhöht sich um die Durchlaßspannung der Gleichrichterdiode; die Betriebsspannung kann durch einen kleinen Glättungskondensator stabilisiert werden, der parallel zum Oszillator liegt und nur durch den Oszillator entladen wird, da die Diode dann in Sperrichtung gepolt ist.

Die Betriebsspannung für die gesamte Schaltung (ohne Modulator und LC Schwingkreis) bleibt dadurch wesentlich stabiler. Dies ist dann besonders wichtig, wenn ohne Spannungsregler gearbeitet wird.

Mit einem Programmiergerät, welches nur einmal während einer Programmierphase an die Sonde angeschlossen wird, wird der spezifische Identifikationscode und die Anzahl der verwendeten Meßsensoren programmiert. Die Sonde wird dabei nur von Programmiergerät aus mit Energie versorgt und nicht über den Hochfrequenzempfangskreis.

Fig 3 zeigt einen Oszillator, der als Fensterkomparator ausgebildet ist, und hierzu zwei Komparatoren Komp1 und Komp2 aufweist. Durch diesen in Fig. 3 dargestellten Aufbau des Oszillators wird eine weitgehende Unabhängigkeit der Frequenz von der Versorgungsspannung V_{cc} sichergestellt. Im übrigen wird zu dem Aufbau des Oszillators auf Fig. 3 verwiesen
Im folgenden wird die Funktionsweise der in Fig. 2 und 3 beschriebenen Schaltung näher erläutert:
Die durch die Auswerteeinheit 3 in Fig. 1 gemessene Frequenz wird ausgewertet und dazu benutzt, die abgestrahlte Leistung der Sendeeinheit 1 einzustellen. Entspricht die gemessene Frequenz der Sollfrequenz f₀ und damit der gewünschten konstanten Betriebsspannung V₀ der Schaltung, so wird der Sender nicht nachgeregelt. Weicht die gemessene Frequenz f von f₀ ab und damit die Betriebsspannung V von V₀ ab, so wird die Sendeleistung solange nachgeregelt, bis die Abweichung zwischen der Frequenz f und f₀ und der Betriebsspannung V und V₀ kleiner ist als ein vorgegebener Wert. Dieser Regelvorgang findet z.B. immer dann statt, wenn sich das Objekt oder Lebewesen, in dem sich der Transponder befindet, bewegt und sich damit der Abstand zwischen Sende-/Empfangseinheit und Transponder variiert. Die abgestrahlte HF-Energie wird durch diesen Regelvorgang immer auf das Minimum eingestellt, welches gerade notwendig ist, um die Schaltung optimal zu betreiben. Dadurch wird auch verhindert, daß die Schaltung durch eine zu hohe Spannung zerstört wird.

Mit Hilfe der oben erwähnten Kennlinie, die den Zusammenhang zwischen Betriebsspannung und Referenzfrequenz darstellt und der abgestrahlten HF-Leistung kann der Abstand zwischen Sende-/Empfangseinheit und Transponder berechnet werden, wenn auch hierfür einmal eine Kennlinie ermittelt wurde.

Die Meßsonde ist miniaturisierbar. Insbesondere ist es möglich, sie so zu miniaturisieren, daß die Gesamtabmessungen der Meßsonde etwa 2 mm im Durchmesser und 10 mm in der Länge betragen. Die integrierte Schaltung hat die Abmessungen von 1,4 mm Breite, 7,4 mm Länge und 0,5 mm Dicke und beinhaltet alle Bauteile, die für die Meßsonde notwendig sind, mit Ausnahme der Spule, des Schwingkreiskondensators und der Gleichrichterdiode. Die Sensoren können prinzipiell mit integriert werden. Die Leistungsaufnahme beträgt bei 1,5V Betriebsspannung weniger als 100 µW. Durch die geringe Leistungsaufnahme kommt die Meßsonde mit einer sehr kleinen Spule von wenigen mm Durchmesser und Länge aus. Die Meßsonde kann somit durch eine Kanüle unter die Haut gespritzt werden.

Dennoch ist der Betriebsspannungsbereich sehr groß und beträgt 1,3 in bis 6 V.

Da die Meßsonde eine Leistung von weniger als 100 µW verbraucht, sind Abstände von der Sende- und Auswerteinrichtung bis in den Bereich von mehreren Metern möglich, ohne daß die Sendeleistungen den Bereich von etwa 1 W überschreiten muß. Dies eröffnet ganz neue Möglichkeiten z.B. bei der Beobachtung von Tieren.

## Patentansprüche

1. Meßsonde mit
- einer Empfangseinheit (3,4), die ein von einer beabstandeten Sende- und Auswerteeinrichtung (5,6) abgestrahltes Hochfrequenzfeld empfängt,
- einer Energieversorgungseinheit, die aus dem hochfrequenten Ausgangssignal der Empfangseinheit eine Versorgungsspannung für die Meßsonde generiert,
- einer Sensoreinheit, die wenigstens einen Sensor (9) aufweist, und deren Ausgangssignale an einen spannungsgesteuerten Oszillator (7) angelegt ist, der das Ausgangssignal in ein Signal umsetzt, dessen Frequenz proportional zum Ausgangssignal ist, und
- einer Modulationseinheit (14), deren Modulationshub programmierbar ist, und die das hochfrequente Ausgangssignal der Empfangseinheit mit dem Ausgangssignal des Oszillators moduliert, und das modulierte hochfrequente Signal an eine Sendeeinheit (1,2) anlegt,
dadurch **gekennzeichnet**, daß die Sensoreinheit zusätzlich wenigstens ein Referenzelement (10) aufweist, dessen Ausgangssignal ein Multiplexer (8) im Multiplexverfahren zu dem Ausgangssignal des oder der Sensoren seriel an den spannungsgesteuerten Oszillator anlegt, wobei das Referenzelement und die zugehörige Schaltung derart gestaltet sind, daß der Einfluß der Betriebsspannung durch eine Änderung der Frequenz des durch das Referenzelement erzeugten Signals ermittelt werden kann, und
daß eine Identifikationseinheit (11,12,13) vorgesehen ist, die einen Identifikationscode erzeugt, mit dem das hochfrequente Signal zur Identifikation der Sonde modulierbar ist, deren Signal jeweils übertragen wird.

2. Meßsonde nach Anspruch 1,
dadurch **gekennzeichnet**, daß mehrere Sensoren vorgesehen sind.

3. Meßsonde nach Anspruch 1 oder 2,
dadurch **gekennzeichnet**, daß die Identifikationseinheit eine Steuerlogik (11,13) und einen Speicher (12) aufweist, in dem die Identifikationscodes gespeichert sind.

4. Meßsonde nach Anspruch 3,
dadurch **gekennzeichnet**, daß eine Programmiereinheit mit der Meßsonde verbindbar ist, um in den Speicher die jeweiligen Identifikationscodes einzugeben.

5. Meßsonde nach einem der Ansprüche 1 bis 4,
dadurch **gekennzeichnet**, daß die Steuereinheit den seriellen Biphase-codierten Datenstrom an den Oszillator überträgt, der ein FSK-Signal generiert.

6. Meßsonde nach einem der Ansprüche 1 bis 5,
dadurch **gekennzeichnet**, daß der Oszillator ein Oszillator mit einem Fensterkomparator ist.

7. Meßsonde nach einem der Ansprüche 1 bis 6,
dadurch **gekennzeichnet**, daß die Empfangs- und die Sendeeinheit denselben Schwingkreis verwenden.

8. Meßsonde nach einem der Ansprüche 1 bis 7,
dadurch **gekennzeichnet**, daß der Modulator direkt an den Schwingkreis angeschlossen ist.

9. Meßsonde nach einem der Ansprüche 1 bis 8,
dadurch **gekennzeichnet**, daß die Sende- und Auswerteeinrichtung eine Regeleinrichtung für die Leistung des abgestrahlten Hochfrequenzfeldes aufweist, an der als Führungsgröße das an einem Referenzelement erhaltene Signal anliegt.

10. Meßsonde nach einem der Ansprüche 1 bis 9,
dadurch **gekennzeichnet**, daß die Sende- und Auswerteeinrichtung den Abstand Meßsonde/Transponder bestimmt.

11. Meßsonde nach einem der Ansprüche 1 bis 10,
dadurch **gekennzeichnet**, daß mehrere Referenzelement vorgesehen sind.

## Claims

1. Measuring probe comprising
- a receiver unit (3, 4) which receives a high-frequency field emitted by a remote transmitter and evaluating means (5, 6),
- a power supply unit which generates a supply voltage for the measuring probe from said high-frequency output signal from said receiver unit,
- a sensor means comprising at least one sensor (9), whose output signal is applied to a voltage-controlled oscillator (7) which converts said output signal into a signal whose frequency is proportional to the output signal, and
- a modulator unit (14) whose modulation depth is programmable and which modulates said high-frequency output signal from said receiver unit with the output signal from said oscillator, and which applies the modulated high-frequency signal to a transmitter unit (1, 2),
**characterized** in that said sensor unit additionally comprises at least one reference element (10) whose output signal is serially applied to said voltage-controlled oscillator by a multiplexer (8) in a process of multiplexing with the output signal from said sensor(s), with said reference element and the appertaining circuit being so designed that the influence of the operating voltage may be determined by a variation of the frequency of the signal generated by said reference element, and that an identification means (11, 12, 13) is provided which generates an identification code by which said high-frequency signal may be modulated for an identification of the probe whose signal is being transmitted.

2. Measuring probe according to Claim 1,
**characterized** in that several sensors are provided.

3. Measuring probe according to Claim 1 or 2,
**characterized** in that said identification means comprises a logic control circuit (11, 13) and a memory (12) where said identification codes are stored.

4. Measuring probe according to Claim 3,
**characterized** in that a programming unit is adapted to be connected to said measuring probe for input of the respective identification codes into said memory.

5. Measuring probe according to any of Claims 1 to 4,
**characterized** in that said control means transmits the serial bi-phase coded data flow to said oscillator which generates an FSK signal.

6. Measuring probe according to any of Claims 1 to 5,
**characterized** in that said oscillator is an oscillator including a window comparator.

7. Measuring probe according to any of Claims 1 to 6,
**characterized** in that said receiver and transmitter units operate on the same oscillatory circuit.

8. Measuring probe according to any of Claims 1 to 7,
**characterized** in that said modulator is connected to said oscillatory circuit directly.

9. Measuring probe according to any of Claims 1 to 8,
**characterized** in that said transmitter and evaluating unit comprises a controller means for controlling the level of the emitted high-frequency field, which the signal obtained at a reference element is applied to as reference variable.

10. Measuring probe according to any of Claims 1 to 9,
**characterized** in that said transmitter and evaluating unit determines the measuring probe/transponder distance.

11. Measuring probe according to any of Claims 1 to 10,
**characterized** in that several reference elements are provided.

## Revendications

1. Tête de mesure qui comprend
- un dispositif récepteur (3, 4) qui reçoit un champ H.F. émis par un dispositif émetteur et d'évaluation (5, 6) éloigné,
- un bloc d'alimentation qui engendre une tension d'alimentation pour la tête de mesure, en la dérivant du signal H.F. de sortie dudit dispositif récepteur,
- un dispositif détecteur qui comprend au moins un détecteur (9), dont le signal de sortie est appliqué à un oscillateur (7) commandé par tension, qui convertit ledit signal de sortie dans un signal dont la fréquence est proportionnelle au signal de sortie, et
- un dispositif modulateur (14) dont l'amplitude de modulation est programmable et qui module ledit signal H.F. de sortie dudit dispositif récepteur avec le signal de sorte dudit oscillateur, et qui applique le signal H.F. modulé audit dispositif émetteur (1, 2),
**caractérisée** en ce que ledit dispositif détecteur de plus comprend au moins un élement de référence (10) dont le signal de sortie est appliqué, de façon sérielle, audit oscillateur commandé par tension, moyennant un multiplexeur (8) au cours d'une opération de multiplexage avec le signal de sortie, qui provient dudit ou desdits détecteur(s), ledit élément de référence et le circuit y affecté étant conçus de façon que l'influence de la tension de régime puisse être détectée par une variation de la fréquence du signal engendré par ledit élément de référence, et
en ce qu'un dispositif d'identification (11, 12, 13) est prévu, qui engendre un code d'identification moyennant duquel ledit signal H.F. peut être modulé pour l'identification de la tête particulière dont le signal est en train d'être transmis.

2. Tête de mesure selon la revendication 1,
**caractérisée** en ce que plusieurs détecteurs sont prévus.

3. Tête de mesure selon la revendication 1 ou 2,
**caractérisée** en ce que ledit dispositif d'identification comprend une logique de commande (11, 13) et une mémoire (12) où lesdits codes d'identification sont mis en mémoire.

4. Tête de mesure selon la revendication 3,
**caractérisée** en ce qu'un dispositif de contrôle de programme est prévu pour être relié à ladite tête de mesure pour l'entrée des codes d'identification respectifs dans ladite mémoire.

5. Tête de mesure selon une quelconque des revendications 1 à 4,
**caractérisée** en ce que ledit dispositif de commande transmet une succession en série de données diphasiques codées vers ledit oscillateur, qui en engendre un signal FSK.

6. Tête de mesure selon une quelconque des revendications 1 à 5,
**caractérisée** en ce que ledit oscillateur est un oscillateur du type qui comprend un comparateur à fenêtres.

7. Tête de mesure selon une quelconque des revendications 1 à 6,
**caractérisée** en ce que lesdits dispositifs récepteur et émetteur fonctionne à la base du même circuit résonnant.

8. Tête de mesure selon une quelconque des revendications 1 à 7,
**caractérisée** en ce que ledit modulateur est relié audit circuit résonnant directement.

9. Tête de mesure selon une quelconque des revendications 1 à 8,
**caractérisée** en ce que ledit dispositif émetteur et d'évaluation comprend un dispositif de commande pour la commande du niveau du champ H.F. émis, auquel est appliqué, comme variable de référence, le signal obtenu à un élément de référence.

10. Tête de mesure selon une quelconque des revendications 1 à 9,
**caractérisée** en ce que ledit dispositif émetteur et d'évaluation détecte la distance entre la tête de mesure et le transpondeur.

11. Tête de mesure selon une quelconque des revendications 1 à 10,
**caractérisée** en ce que plusieurs éléments de référence sont disposés.
